(19) Europäisches Patentamt
European Patent Office
Office européen des brevets

(11) **EP 1 832 556 A1**

(12) **EUROPÄISCHE PATENTANMELDUNG**

(43) Veröffentlichungstag:
**12.09.2007 Patentblatt 2007/37**

(51) Int Cl.:
*C02F 3/12* (2006.01)     *C02F 3/30* (2006.01)

(21) Anmeldenummer: **06004586.1**

(22) Anmeldetag: **07.03.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Aqua Service Schwerin Beratungs- und Betriebsführungsgesellschaft mbH 19061 Schwerin (DE)**

(72) Erfinder: **Jagnow, Bert 19059 Schwerin (DE)**

(74) Vertreter: **Seemann, Ralph Patentanwälte Seemann & Partner, Ballindamm 3 20095 Hamburg (DE)**

(54) **Verfahren zum Betreiben einer biologischen Kläranlage**

(57) Die Erfindung betrifft ein Verfahren zum Betreiben einer biologischen Kläranlage (1) mit wenigstens einem Belebtschlammbecken (2) sowie ein Computerprogramm mit Programmcode-Mitteln, die angepasst sind, um das Verfahren auszuführen.

Das erfindungsgemäße Verfahren zeichnet sich dadurch aus, dass die Häufigkeiten mehrerer Arten von im Belebtschlammbecken (2) enthaltenen Mikroorganismen gemessen wird und aus den Messergebnissen wenigstens eine Zustandskenngröße der Kläranlage (1) ermittelt wird.

Fig. 1

EP 1 832 556 A1

## Beschreibung

Die Erfindung betrifft ein Verfahren zum Betreiben einer biologischen Kläranlage mit wenigstens einem Belebtschlammbecken sowie ein Computerprogramm mit Programmcode-Mitteln, die angepasst sind, um das Verfahren auszuführen.

In biologischen Kläranlagen wird zur Abwasserreinigung häufig das Belebtschlammverfahren verwendet. Dabei wird Abwasser durch die biologische Aktivität von Mikroorganismen in einem Belebtschlamm weitestgehend von organischen Verunreinigungen gereinigt. Als Belebtschlamm wird eine Ansammlung von Mikroorganismen, die unter aeroben, d.h. sauerstoffreichen, Bedingungen organische kohlenstoffhaltige Stoffe abbauen, bezeichnet. Der Belebtschlamm besteht vor allem aus Bakterien, Pilzen und Protozoen. Bestimmte Mikroorganismen Im Belebtschlamm sorgen darüber hinaus für die Verwertung des in der Biomasse enthaltenen Phosphors, Stickstoffes und anderer Elemente.

Kläranlagen, die nach dem Belebtschlammverfahren arbeiten, sind sowohl im Durchlaufbetrieb als auch Im diskontinuierlichen Batch-Betrieb bekannt.

Im Durchlaufbetrieb wird das Gemisch aus zu reinigendem Abwasser und reinigungsaktivem Belebtschlamm im Belebtschlammbecken sowohl durch Sauerstoffeintrag als auch optional durch mechanische Verwirbelungsmittel ständig durchmischt, um eine optimale Reinigung des Wassers zu erreichen.

Aus dem Belebtschlammbecken fließt das Gemisch aus Wasser und Belebtschlamm in ein Nachklärbecken. Der Belebtschlamm setzt sich am Beckenboden des Nachklärbeckens durch Sedimentation ab und wird dort durch sein Eigengewicht eingedickt. Zur Unterstützung der Sedimentation im Nachklärbecken wird dort ein sehr langsamer Fluss des Wassers eingestellt und kein Sauerstoff eingetragen. Der sedimentierte Belebtschlamm wird vom Beckenboden abgezogen, teilweise als Rücklaufschlamm in das Belebungsbecken zurückgeführt und anderenteils in Faultürmen weiter verarbeitet. Das gereinigte und von Belebtschlamm weitgehend befreite Abwasser verlässt das Nachklärbecken über ein Überlaufwehr.

Im Batch-Betrieb wird in einem Becken unter Sauerstoffeintrag und mechanischer Verwirbelung das zu reinigende Abwasser durch den Belebtschlamm gereinigt. Nachfolgend wird der Sauerstoffeintrag und die Verwirbelung gestoppt, so dass der Belebtschlamm sich absetzen kann. Nach vollständiger Sedimentation wird dann das gereinigte Wasser aus dem Becken entleert, gegebenenfalls überschüssiger Belebtschlamm entfernt und ein weiterer Reinigungszyklus durch Zugabe von zu reinigendem Wasser eingeleitet.

In belden Betriebsarten liegt der Belebtschlamm in Form von Belebtschlammflocken vor. Die Belebtschlammflocken sind Kolonien von Mikroorganismen und mit bloßem Auge erkennbar. Die in und auf den Belebtschlammflocken angesiedelten Mikroorganismen gehören in der Regel verschiedenen Arten an, die auf den Abbau verschiedener Schmutzstoffe spezialisiert sind. So kann ein breit gefächertes Spektrum von Schmutzstoffen im Abwasser abgebaut werden.

Die meisten Mikroorganismen im Belebtschlamm benötigen für ihre Aktivität Sauerstoff. Es muss daher ein genügender Sauerstoffeintrag In das Belebtschlammbecken sichergestellt werden. Die Sauerstoffversorgung geschieht üblicherweise durch Einblasen von Druckluft vom Beckenboden, durch Oberflächenbelüfter oder durch Begasung mit reinem Sauerstoff. Bei Sauerstoffmangel kann der Belebtschlamm faulen, die Reinigungswirkung der biologischen Kläranlage lässt nach.

Um sicherzustellen, dass die eingetragene Menge des Sauerstoffs sowie dessen gleichmäßige Verteilung Im gesamten Volumen des Belebtschlammbeckens ausreichend ist, wird üblicherweise der Sauerstoffverbrauch anhand des biologischen bzw. biochemischen Sauerstoffbedarfs ($BSB_5$) gemessen. Der $BSB_5$ ist die Menge an Sauerstoff in mg/l, den Bakterien und andere im Wasser vorhandene Mikroorganismen bei einer Temperatur von 20°C innerhalb von fünf Tagen zum Abbau von biologisch abbaubaren, organischen Stoffen verbrauchen und ist somit ein Indikator für den Grad der Verschmutzung des Abwassers. Der $BSB_5$ beträgt in der Regel etwa 70 % des gesamten biologischen Sauerstoffbedarfs. Eine Messunsicherheit zum $BSB_6$ besteht darin, dass weiterer Sauerstoffbedarf durch andere Sauerstoff konsumierende Prozesse, wie beispielsweise Nitrifikation, die Umwandlung organisch gebundenen Stickstoffs in Nitrat entsteht.

Im Belebtschlamm sind Mikroorganismen verschiedener Arten auf verschiedene Umgebungsbedingungen angepasst und wirken als Katalysatoren für verschiedene chemische Reaktionen, in denen organische und anorganische chemische Verbindungen aufgebrochen und umgewandelt werden. Beispielsweise wandeln so genannte Nitrifikanten Stickstoff, der in organischen Verbindungen vorkommt, und, wenn er ungefiltert ins Frischwasser gelangt, zur Eutrophierung von Gewässern beiträgt, zu Nitrat um. Dabei werden durch langsam wachsende Bakterien, die Nitrifikanten, Ammoniak-Moleküle ($NH_3$) schrittweise zu Nitrit ($NO_2^-$) und nachfolgend zu Nitrat ($NO_3^-$) oxidiert, wobei außerdem $H^+$-Ionen gebildet werden, die zu einer Versäuerung des Abwassers führen können. Typische Nitrifikanten sind beispielsweise die Bakterien der Familien Nitrobacter, Nitrosomonas, Nitrosococcus u.a.

Andere Bakterien (Denitrifikanten, beispielsweise Pseudomonas denitrificans, Thiobacillus denitrlflcans, Paracoccus denitrificans) wandeln die Nitrate unter Sauerstoffabschluss, also unter anaeroben Bedingungen, unter schrittweisem Sauerstoffentzug zu molekularem Stickstoff um, der aus dem Abwasser In die Atmosphäre entweicht (Denitrifikation). Dabei werden $H^+$-Ionen absorbiert, das pH-Gleichgewicht wird wieder hergestellt. Durch intermittierenden Betrieb, durch Ein- und Ausschalten der Belüftung oder durch Anordnen von abwechselnden sauerstoffreichen und -armen Zonen in einem durchströmten Becken können Denitrifikation und Nitrifikation Im gleichen Becken ablaufen. Bei Anlagen mit wenigstens zwei Becken kann die Denitrifikation der Nitrifikation vor- oder nachgeschaltet sein.

Die Entfernung von Phosphat aus dem Abwasser geschieht In herkömmlichen Kläranlagen durch Einsatz von Fällmitteln, nämlich Metallsalzen, die mit dem Phosphat schwerlösliche Verbindungen eingehen, die ausfallen und sich absetzen. Es gibt jedoch auch Mikroorganismen im Belebtschlamm biologischer Kläranlagen, die Phosphat biologisch eliminieren. Beispielsweise wandeln Mykorrhiza-Bakterien Phosphat in Polyphosphat-Granulat um, das sie in ihre Biomasse aufnehmen. Auch hier wird jedoch optional Fällmittel zur zusätzlichen Ausfällung von Phosphat zugegeben.

Eine typische Kenngröße einer biologischen Kläranlage ist neben dem biologischen Sauerstoffbedarf $BSB_5$ die Schlammbelastung $B_{TS}$. Die Schlammbelastung gibt die Masse an $BSB_5$ an, die pro Tag einem Kilogramm Trockensubstanz (TS) zugeführt wird. Dabei ist die Trockensubstanz die Substanz des getrockneten Belebtschlammes. Ist die Schlammbelastung zu hoch, kann der Belebtschlamm die Masse an biologischen Stoffen, die abzubauen sind, nicht mehr bewältigen und es muss weitere Belebtschlamm-Trockensubstanz zugeführt werden. Ist die Schlammbelastung zu niedrig, findet eine Unterversorgung des Belebtschlamms mit Nahrungsstoffen statt, so dass Belebtschlamm abgeführt werden muss.

Bei biologischen Kläranlagen nach dem Belebtschlammverfahren mit Durchlaufbetrieb setzt sich in der Nachklärung zwar der größte Teil des Belebtschlamms als Sediment am Boden des Nachklärbeckens ab, wird teilweise in das Belebtschlammbecken zurückgeführt und teilweise in Faultürme überführt. Ein kleiner Teil des Belebtschlamms verlässt als Ablauf jedoch das Nachklärbecken zusammen mit dem gereinigten Wasser, vor allem, wenn sich so genannter Blähschlamm bildet, bei dem fadenförmige Mikroorganismen Belebtschlammflocken zu extrem leichten Gebilden vereinigen. Dadurch werden deren Sedimentationseigenschaften so verschlechtert, dass leichte Flockenverbände auf der Oberfläche des gereinigten Wassers schwimmen und durch den Überlauf für das gereinigte Wasser massenhaft aus dem Nachklärbecken austreten. Dies ist der so genannte Schlammabtrieb.

Durch Schlammabtrieb sinkt der Biomassegehalt im Kläranlagen-System, die Ablaufwerte verschlechtern sich und es nimmt die Reinigungsleistung ab. Häufige Ursachen hierfür sind Nährstoffmangel und leicht abbaubares oder angefaultes Abwasser, beispielsweise aus der Lebensmittelindustrie. Durch die Zugabe von Fällmitteln, die den Blähschlamm beschweren, durch Verkürzung der Aufenthaltsdauer im Vorklärbecken oder durch Zugabe von Nährstoffen und Änderungen der Verfahrensführung können die Ursachen und Auswirkungen von Blähschlamm bekämpft werden.

Der Belebtschlamm biologischer Kläranlagen bildet ein biologisches System, das auf Betriebsbedingungen, wie u. a. die Menge des zuströmenden Wassers, Zusammensetzung des Abwassers und seine Belastung mit organischen und anorganischen Inhaltsstoffen, Störungen in der Sauerstoffversorgung usw., reagiert. Die Reinigungsleistung einer biologischen Kläranlage kann innerhalb kurzer Zeit aufgrund äußerer Einflüsse zu- oder abnehmen. Darum wird der Betriebszustand von biologischen Kläranlagen durch die Auswertung von physikalisch-chemischen Wasser- und Schlammkenngrößen überwacht. Diese erlauben Rückschlüsse auf die Sauerstoffversorgung, Schlammbelastung und die Stabilität der Nitrifikation, der Sedimentation des Schlammes im Nachklärbecken und des biologischen Sauerstoffbedarfs im Ablauf der Kläranlage.

Zusätzlich wird auch die Untersuchung des mikroskopischen Bildes, nämlich Struktur und Größe der Belebtschlammflocken, Fädigkeit und Schlammfarbe, zur Bewertung des Betriebszustandes des Belebtschlammes einer Kläranlage hinzugezogen. Diese lassen eine allgemeine Einschätzung des Betriebszustandes des Belebtschlammes zu, jedoch keine aussagefähigen Zahlen, die mit den Messergebnissen der chemischen und physikalischen Wasseranalytik vergleichbar sind. Mit diesen Messungen sind Veränderungen, die sich kurz- oder mittelfristig auf die Ablaufwerte der Kläranlage auswirken, wie z.B. eine steigende Schlammbelastung, eine Verschlechterung der Sauerstoffverhältnisse oder auch Veränderung in der Schlammbeschaffenheit, sowie eine zu erwartende Überschreitung von Grenzwerten für die Ablaufwerte, also der im gereinigten Wasser enthalten Verunreinigungen, nicht immer rechtzeitig erkennbar.

Der Erfindung liegt daher die Aufgabe zugrunde, ein Verfahren zum Betreiben einer biologischen Kläranlage zur Verfügung zu stellen, mittels dessen besser auf allmähliche oder kurzfristige Veränderungen der Betriebsbedingungen reagiert werden und mittels dessen eine Einhaltung von Grenzwerten für die Ablaufwerte der Kläranlage dauerhaft sichergestellt wird.

Die Aufgabe wird durch ein Verfahren zum Betreiben einer biologischen Kläranlage mit wenigstens einem Belebtschlammbecken gelöst, das dadurch weitergebildet ist, dass die Häufigkeit mehrerer Arten von im Belebtschlammbecken enthaltenen Mikroorganismen gemessen wird und aus den Messergebnissen wenigstens eine Zustandskenngröße der Kläranlage ermittelt wird. Die Messungen können auch in gegebenenfalls ebenfalls vorhandenen Nachklärbecken zusätzlich vorgenommen werden oder anstelle der Messungen des Inhalts des Belebtschlammbeckens.

Die Erfindung beruht auf dem grundlegenden Gedanken, dass eine neuartige und verbesserte Analytik zusätzlich oder alternativ zur bekannten physikalischen-chemischen Analytik und der rein qualitativen Bewertung der Schlammeigenschaften der biologischen Kläranlage eine verbesserte und frühzeitige Reaktion auf sich verändernde Bedingungen in der Kläranlage ermöglicht. Dabei wird die Tatsache ausgenutzt, dass die verschiedenen, Im Belebtschlamm und in der Wasserphase enthaltenen Arten von Mikroorganismen mehr oder weniger spezifisch an bestimmte Umgebungsbedingungen angepasst sind und daher jeweils eine mehr oder weniger starke Indikatorfunktion für eben diese Umgebungsbedingungen haben. Die Artenzusammensetzung kann sehr kurzfristig, d. h. innerhalb weniger Stunden, auf Änderungen der Bedingungen im Belebtschlammbecken reagieren.

Die Messung der Häufigkeiten der im Belebtschlamm vertretenen Arten von Mikroorganismen lässt bei Kenntnis dieser Indikatoreigenschaften eine zuverlässige und schnelle Bestimmung der im Belebtschlammbecken bzw. der Kläranlage herrschenden Bedingungen zu.

Eine wesentlich eingeschränkte Analytik auf der Grundlage eines Artenspektrums ist bislang nur für die Ermittlung des biologischen Verschmutzungsgrades von Fließgewässern und Seen und deren Einordnung in Gewässergüterklassen In Form des Saproblensystems bekannt. Die Saprobität ist dabei ein Maß für die Bioaktivität des Gewässers und rangiert von der Katharobität, in der fast keine Saprobien (Mikroorganismen) vorhanden sind, wie bei Grundwasser mit Trinkwasserqualität, bis zur Ultrasaprobität, bei der biologische Aktivität nicht mehr möglich ist. Die Katharobität wird mit einem Saprobienindex von 1 gleichgesetzt, die Ultrasaprobität mit einem Saprobienindex von 4.

Von ökologischer Bedeutung ist die Limnosaprobität (Saprobienindex ca. 2). Innerhalb der Limnosaprobität, also bei sauberen bis verunreinigten Oberflächengewässern, werden Güteklassen I bis IV von unbelastet bis übermäßig verschmutzt aufgrund von 48 Leitsaprobien (Zelgerorganismen) bewertet. Der Saprobienindex für Oberflächengewässer wird anhand der Häufigkeltsstufen $A$ (Abundanz zwischen 1 für Einzelfunde und 7 für massenhaftes Auftreten) der Zeigerorganismen, ihrem Saprobienwert s (zwischen 1 und 4) und einem Indikationsgewicht $g$ (zwischen 1 und 16) berechnet. Das Indikationsgewicht bezeichnet den Wert einer Art als Indikator, wobei einer toleranten Art ein niedriges Indikationsgewicht von 1 und einer sehr speziell angepassten Art ein hohes Indikationsgewicht von bis zu 16 zugewiesen wird. Der Saprobienindex S berechnet sich nach DIN 38410 nach der Formel

$$S = \frac{\sum_{i=1}^{n} A_i \cdot s_i \cdot g_i}{\sum_{i=1}^{n} A_i \cdot g_i},$$

als gewichtete Summe über die Saprobienwerte $s_l$ der Arten der Zeigerorganismen, wobei n für die Anzahl der vorgefundenen Arten steht.

Dieses System ist jedoch auf biologische Kläranlagen nur begrenzt übertragbar, weil die Wasserqualität wesentlich schlechter ist als In den meisten Fließgewässern. Belebte Abwässer haben eine Eusaprobität mit einem Saprobienindex zwischen 2,5 und 3,5. Viele Zeigerorganismen für Oberflächengewässer sind an die Bedingungen in biologischen Kläranlagen nicht oder kaum angepasst und kommen dort auch nicht vor.

Im Rahmen der Erfindung werden daher die Zeigerorganismen für den Saprobienindex für Oberflächengewässer durch für Belebtschlämme bzw. Wasserphasen in biologischen Kläranlagen typische. Zeigerorganismen ergänzt. So lassen sich Zustandskenngrößen der Kläranlage in zum Saprobiensystem analoger Welse In einem quantitativen numerischen System ermitteln und gegebenenfalls notwendige Änderungen der Einstellungen des Klärbetriebs bestimmen.

Wenn vorzugsweise zusätzlich wenigstens eine Kenngröße zur Beschaffenheit eines Inhalts des wenigstens einen Belebtschlammbeckens gemessen wird, ist eine noch genauere und einfache Bestimmung des Zustands der Kläranlage und des Belebtschlamms möglich. Daraus ergibt sich eine noch bessere Grundlage zur Bewertung, welche Aktionen Im Betrieb der Kläranlage vorgenommen werden müssen.

Vorzugsweise werden als Kenngröße zur Beschaffenheit des Inhalts des Belebtschlammbeckens eine Farbe und/oder ein Geruch gemessen. Die Farbe, beispielsweise braun - beige - grau - schwarz, sowie der Geruch, beispielsweise erdig - muffig - stechend - faulig, geben Aufschluss beispielsweise darüber, ob eine ausreichende Sauerstoffversorgung vorhanden ist oder ob das Alter des Schlamms möglicherweise zu niedrig ist.

Weiter vorzugsweise wird als Kenngröße eine Opazität einer Wasserphase des Inhalts des wenigstens einen Belebtschlammbeckens und/oder eine Häufigkeit frei lebender Bakterien in der Wasserphase gemessen. Dabei ist die Wasserphase ein Teil des zu reinigenden Abwassers ohne Belebtschlammflocken. Daher betreffen diese Kenngrößen nicht die auf den Belebtschlammflocken angesiedelten Kolonien von Mikroorganismen, sondern Schwebstoffe und frei lebende Bakterien in der Wasserphase. Diese Mikroorganismen sind beispielsweise Indikatoren für eine gute Sauerstoffversorgung.

Weiterhin bevorzugt wird als Kenngröße eine Fädigkeit eines im wenigstens einen Belebtschlammbekken enthaltenen Belebtschlamms und/oder eine Größe und/oder eine Struktur von Belebtschlammflocken des Belebtschlamms gemessen. Die Fädigkeit des Belebtschlamms lässt Rückschlüsse darauf zu, wie wahrscheinlich ein vermehrter Schlammabtrieb im Ablauf des Nachklärbeckens oder des im Batch-Modus betriebenen Belebtschlammbeckens ist.

Die Struktur der Belebtschlammflocken lässt ebenfalls Rückschlüsse auf das Sedimentationsverhalten des Belebtschlamms zu. So ist eine feste, kompakte und abgerundete Flockenstruktur ein Indikator für eine gute Sedimentation. Andere Flockenstrukturen sind beispielsweise unregelmäßig, lappig - locker, vernetzt - dendritenartig. Diese Strukturen haben fortschreitend schlechtere Sedimentationseigenschaften.

Vorteilhafterweise wird als Zustandskenngröße der biologischen Kläranlage eine Sauerstoffversorgung und/oder ein Sauerstoffversorgungsindex und/oder ein Saprobienindex und/oder eine Schlammbelastung und/oder eine Schadstoffbelastung und/oder eine Ablaufkonzentration und/oder eine Nitrifikationskonngröße ermittelt. Jede dieser Kenngrößen gibt Auskunft über bestimmte Aspekte des Betriebszustands der Kläranlage. Dabei reicht beispielsweise die Bewertung der Sauerstoffversorgung bzw. des Sauerstoffversorgungsindex von sehr gut bis Fäulnis bzw. deren numerischen Äquivalenten. Ein Saprobienindex, der gegenüber einem Saprobienindex für Fließgewässer um Indikatororganismen aus dem Belebtschlamm bereichert ist, vermittelt u. a. Informationen über die Stabilität des Betriebs der Kläranlage. Die Schlammbelastung vermittelt Informationen darüber, ob der Belebtschlamm über- oder unterversorgt ist, und ob dementsprechend Belebtschlamm-Trockensubstanz zugeführt oder abgezogen werden muss.

Bestimmte Mikroorganismen sind auf verschiedene Schadstoffe sensitiv, so dass eine Schadstoffbelastung und ggf. die Notwendigkeit einer weiteren, insbesondere chemischen Reinigung, erkennbar ist. Ebenfalls lässt sich aus den Kenngrößen auf eine Ablaufkonzentration von ungereinigten biologischen und anderen Stoffen schließen, wobei bei deren Anwachsen die Effektivität der Kläranlage erhöht werden muss. So sind beispielsweise bestimmte Amöben und bestimmte Rotatoria (Rädertierchen) Indikatoren für eine niedrige, bestimmte Streptokokken und Ciliaten (Wimperntierchen) Indikatoren für eine hohe Ablaufkonzentration biologischer Reststoffe.

Indikatororganismen für eine gute Sauerstoffversorgung sind beispielsweise Tardigrada (Bärtierchen), während bestimmte Trepomonas- und Trigonomes-Flagellaten Sauerstoffmangel anzeigen. Bärtierchen zeigen auch eine niedrige Schlammbelastung an, wie auch einige Rotatorien (Rädertierchen), während Zoogloea-Kolonlen und bestimmte Streptokokken eine hohe Schlammbelastung Indizieren.

Der zu erwartende Schlammabtrieb wird u. a. aus der gemessenen Fädigkeit, Größe und Struktur der Belebtschlammflocken sowie anderen charakteristischen Größen ermittelt. Die Nitrifikation ist beispielsweise durch vermehrtes Vorhandensein von höheren Organismen, wie Borstenwürmer und Rädertieren, indiziert.

Vorzugsweise werden zum Ermitteln von Zustandskenngrößen der Kläranlage den Messergebnissen Gewichtungen, insbesondere für ihre jeweilige Indikatoreigenschaft für bestimmte Betriebszustände der Kläranlage, zugewiesen. Zum Ermitteln von Zustandskenngrößen der Kläranlage werden vorzugsweise die Produkte aus den Messergebnissen und den ihnen zugewiesenen Gewichtungen addiert.

Dies geschieht insbesondere nach der oben angegebenen allgemeinen Formel zum Saprobienindex, wobei jedoch statt der Saprobität S und den für die einzelnen Arten spezifischen Indizes $s_l$ und Toleranzfaktoren $g_i$ die jeweiligen Indizes und kenngrößenspezifischen Toleranzfaktoren der Zeigerorganismen des Belebtschlammes und der Wasserphase für die zu ermittelnden Kenngrößen der Kläranlage einzusetzen sind. Beispielsweise berechnen sich Zustandskenngrößen $K_j$ (beispielsweise $BSB_5$, $B_{TS}$, Nitrifikation etc.) bei n vorgefundenen Zeigerorganismen als

$$K_j = \frac{\sum_{i=1}^{n} A_i \cdot k_{j,i} \cdot g_{j,i}}{\sum_{i=1}^{n} A_i \cdot g_{j,i}},$$

wobei die Abundanz $A_i$ der Arten von Mikroorganismen natürlich für alle Kenngrößen gleich ist, während die Indizes $k_{j,i}$ und Toleranzwerte $g_{j,i}$ der Mikroorganismen für jede Kenngröße $K_j$ unterschiedlich sein kann. Eine Zustandskenngrößenbestimmung kann auch lediglich vom Zähler der genannten Formel Gebrauch machen, beispielsweise indem zu jeder Stufe einer Kenngröße die im Zähler genannte Summe über die für diese Stufe typischen Zeigerorganismen gebildet wird und die Summen für die verschiedenen Stufen miteinander in Beziehung gesetzt oder verglichen werden.

In die Bestimmung der Zustandskenngrößen der Kläranlage fließen vorteilhafterweise mikroskopische Messergebnisse, wie Farbe, Geruch, Flockengrößen usw., sowie insbesondere physikalisch-chemische Messergebnisse ein, Es ergibt sich auf diese Weise eine sehr genaue Charakterisierung des Betriebszustandes der biologischen Kläranlage.

Vorteilhafterweise wird das Ermitteln von Zustandskenngrößen der Kläranlage wiederholt, So werden in zeitlicher Abfolge Messreihen aufgebaut, die insbesondere auf Veränderungen im Betriebszustand schließen lassen. Auf die Veränderungen wird dann in entsprechender Weise reagiert, um einen besseren oder optimalen Betriebszustand herzustellen.

Wenn vorteilhafterweise aufgrund wenigstens einer ermittelten Zustandskenngröße oder aufgrund einer Änderung wenigstens einer ermittelten Zustandskenngröße wenigstens eine Betriebskenngröße der Kläranlage verändert wird, ist es möglich, auf die Kläranlage einzuwirken und sie zu einem gewünschten, insbesondere für die Kläranlage optimalen Zustand, zu steuern bzw. zu regeln.

Als Betriebskenngröße der Kläranlage wird vorteilhafterweise eine Sauerstoffeintragung und/oder eine Eintragung einer Trockensubstanz In das wenigstens eine Belebtschlammbecken und/oder eine Fällmittelzugabe In das wenigstens eine Belebtschlammbecken und/oder in wenigstens ein dem wenigstens einen Belebtschlammbecken nachgeschalteten Nachklärbecken verändert. So bewirkt die Reduzierung des Abzuges von Belebtschlammkulturen eine Reduzierung der Schlammbelastung und es wird die Nitrifikation verbessert. Mittelbar werden so auch die Ablaufwerte verbessert. Eine Erhöhung der Sauerstoffversorgung führt zu einem besseren Abbau von Inhaltsstoffen des Abwassers, die Nitrifikation und die Ablaufwerte werden verbessert. Bei vermehrtem Schlammabtrieb wird beispielsweise eine Fällmittelzugabe im Nachklärbecken erhöht, um Sedimentation auch des Blähschlamms zu erreichen. Fällmittelzugabe ist auch beispielsweise im Belebtschlammbecken angebracht, wenn erhöhte Abtriebwerte von Phosphor oder anderen Inhaltsstoffen, die auch durch Fällung zu reduzieren sind, gemessen werden.

Eine besonders bevorzugte Weiterbildung des erfindungsgemäßen Verfahrens besteht darin, dass in aufeinanderfolgenden Iterationen des Ermittelns von Zustandsgrößen und/oder des Veränderns von Betriebskenngrößen der Kläranlage die Gewichtung der Messergebnisse und/oder das Ausmaß der Veränderungen von Betriebskenngrößen, insbesondere in einem Selbstlernprozess, optimiert wird.

Dadurch, dass durch Veränderung von Betriebskenngrößen der Kläranlage, also beispielsweise Erhöhung des Sauerstoffeintrags, Zugabe von Trockensubstanz zum Belebtschlamm, Erhöhung der Fällmittelzugabe, die Messergebnisse verändert werden, lässt sich im Laufe der Zeit mit zunehmender Sicherheit für eine bestimmte Kläranlage beurteilen, welche Mikroorganismen Zeigerorganismen für bestimmte Betriebszustände sind und was ihre Toleranz dafür ist. Das gleiche gilt auch für die allgemeinen Kenngrößen, wie Schlammfarbe, -geruch, usw. Insofern wird für jedes Belebtschlammbecken bzw. für jede Kläranlage ein für die jeweilige Anlage charakteristisches optimales Profil von Indikatororganismen, deren Gewichtungen und spezifischen Toleranzen erzeugt, ausgehend von einem Standardprofil, in dem typische Indikatororganismen mit ihren Gewichtungen und Toleranzen verzeichnet sind.

Durch die Unterschiedlichkeit, beispielsweise der regionalen Wasserhärte und der zu verarbeitenden Schmutzstoffe, etwa abhängig davon, ob eine Kläranlage vermehrt landwirtschaftliche oder gewerbliche Abwässer verarbeitet, können sich regionale Unterschiede in den Artenspektren und Gewichtungen ergeben, so dass diese von Kläranlage zu Kläranlage deutlich variieren können.

Die erfindungsgemäßen Messungen sowohl der allgemeinen Parameter als auch der Häufigkeiten der im Belebtschlamm und in der Wasserphase vorkommenden Arten von Mikroorganismen ist durch angelernte Personen einfach, schnell und zuverlässig ausführbar. In einer vorteilhaften Weiterbildung wird darüber hinaus die Messung der Häufigkeit wenigstens einer Art von Im Belebtschlammbecken enthaltenen Mikroorganismen und/oder die Messung einer weiteren Kenngröße zur Beschaffenheit eines Inhalts des wenigstens einen Belebtschlammbeckens automatisch vorgenommen, insbesondere mittels eines Bilderkennungsverfahrens. Das Bilderkennungsverfahren basiert vorteilhafterweise auf einem selbstlernfähigen Verfahren, insbesondere einem neuronalen Netz, das dazu trainiert wird, im Belebtschlammbecken vorkommende Mikroorganismen zu identifizieren. Ein entsprechendes Verfahren und eine Vorrichtung zum Erstellen einer Artenliste für eine flüssige Probe ist beispielsweise aus DE 42 44 708 C2 bekannt. Auf diese Weise ist eine reproduzierbare und zuverlässige Häufigkeitsmessung und Identifikation der Mikroorganismen im Belebtschlammbecken möglich und eine zuverlässige Ermittlung des Betriebszustandes der Kläranlage.

Weiterhin wird die der Erfindung zugrunde liegende Aufgabe gelöst durch ein Computerprogramm mit Programmcode-Mitteln, die angepasst sind, um das erfindungsgemäße Verfahren auszuführen. Das betrifft sowohl das Verfahren, bei dem die Messungen ganz oder teilweise durch Personen durchgeführt werden als auch das Verfahren, bei dem die Messungen ausschließlich automatisch durch Vorrichtungen oder Computer vorgenommen werden. Weiterhin wird die der Erfindung zugrunde liegende Aufgabe gelöst durch ein Computerprogramm wie vorstehend beschrieben, das auf einem von einem Computer lesbaren Datenträger gespeichert ist.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1 eine schematische Darstellung eines Teils einer biologischen Kläranlage und

Fig. 2 eine schematische Darstellung des Ablaufs des erfindungsgemäßen Verfahrens.

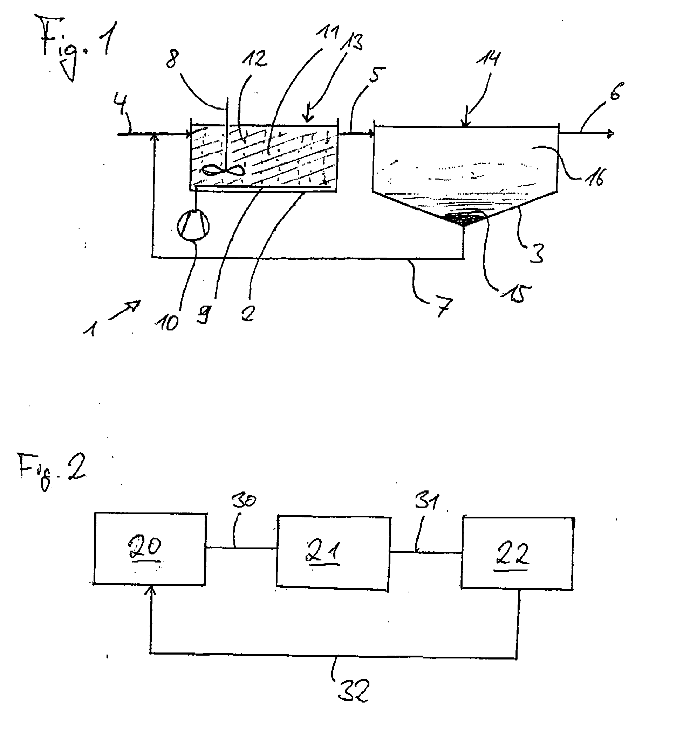

In Fig. 1 ist eine biologische Kläranlage 1 in schematischer Darstellung gezeigt. Die biologische Kläranlage 1 weist ein Belebtschlammbecken 2 und ein Nachklärbecken 3 auf, wird also im Durchlaufbetrieb betrieben. Ein Zulauf 4 führt zu reinigendes Abwasser mit organischen und anderen Inhaltsstoffen dem Belebtschlammbecken 2 mit Belebtschlamm zu, der für die Reinigung und Nitrifikation der Abwasserinhaltsstoffe sorgt. Im Belebtschlammbecken sorgt ein Rührer 8 für die gleichmäßige Durchmischung des Abwassers mit dem Belebtschlamm. Mittels eines Sauerstoffverteilers 9 wird von einer Druckluftquelle 10 bereit gestellte Luft In das Belebtschlammbecken eingetragen und so Sauerstoff in Luftblasen 12 im Abwasser-Belebtschlamm-Gemisch 11 verteilt. Durch einen Pfeil ist ein Fällmitteleintrag 13 dargestellt, der zur zusätzlichen Ausfällung von Phosphor oder anderen im Abwasser vorhandenen Inhaltsstoffen zugegeben wird.

Über einen Überlauf 5 gelangt das Abwasser-Belebtschlamm-Gemisch 11 in ein Nachklärbecken 3, in dem das gereinigte Wasser 16 sehr langsam und ungestört fließt. Daher setzt sich am Grund des Nachklärbeckens 3 Belebtschlamm in einer Sedimentationsschicht 15 ab. An der Oberfläche des Nachklärbeckens 3 bleibt gereinigtes Wasser 16 zurück. Zur Verhinderung von Schlammabtrieb, zur Unterstützung der Sedimentation des Belebtschlammes und zur Fällung von einmal entstandenem Blähschlamm wird Fällmittel 14 in das Nachklärbecken 3 eingetragen. Das gereinigte Wasser tritt über einen Ablauf 6, der auch ein Überlauf ist, aus dem Nachklärbecken 3 aus.

Am Boden des Nachklärbeckens 3 wird der sedimentierte Belebtschlamm 15 abgepumpt und in einer Belebtschlammrückführung 7 zum Zulauf 4 des Belebtschlammbeckens 2 zurückgeführt. Nicht dargestellt ist, dass ein Teil des aus dem Nachklärbecken 3 abgepumpten Belebtschlammes in einen Faulturm überführt wird, um zu Dünger weiterverarbeitet zu werden. Ebenfalls nicht dargestellt sind typische Klärmittelanlagenbestandteile, wie Rechen, Sandbecken, Zwischenklärbecken usw. Für das erfindungsgemäße Verfahren werden in regelmäßigen Abständen Proben aus dem Belebtschlammbecken 2 und/oder aus dem Nachklärbecken 3 entnommen und analysiert. Diese Proben sowie die Probenentnahme sind in Fig. 1 nicht dargestellt.

In Fig. 2 Ist ein Ausführungsbeispiel eines Ablaufs des erfindungsgemäßen Verfahrens schematisch dargestellt. In einem Schritt 20 werden Proben aus dem Belebtschlammbecken 2 und/oder dem Nachklärbecken 3 entnommen und Häufigkeiten von Mikroorganismen im Belebtschlamm und in der Wasserphase bestimmt. Ebenso werden weitere allgemeine Zustandsgrößen, wie Opazität, Farbe, Geruch usw. bestimmt. Aus diesen Messgrößen werden im Schritt 30 Zustandskenngrößen der Kläranlage 1 ermittelt, die in einem Schritt 21 dazu dienen, den Betriebszustand der Kläranlage 1 zu ermitteln.

In einem Schritt 31 wird ermittelt, welche Änderungen von Betriebskenngrößen der Kläranlage, also Sauerstoffversorgung, Fällmittel- oder Trockensubstanzzugabe usw. nötig ist, um einen optimalen Betriebszustand der Kläranlage 1 zu erreichen. Diese Änderung der Betriebskenngrößen der Kläranlage 1 wird im Schritt 22 durchgeführt. Diese geänderten Betriebskenngrößen der Kläranlage 1 haben im nachfolgenden Schritt 32 Auswirkungen auf die Mikroorganismen im Belebtschlamm im Belebtschlammbecken 2 und auf andere Messgrößen.

Diese Auswirkungen finden auf sehr verschiedenen Zeitskalen statt. So ist die Lebensdauer der einzelnen Mikroorganismenarten sehr unterschiedlich und kann von einer Stunde bis zu einem halben Tag betragen, wobei beispielsweise die Nitrifikationsorganismen und die Fadenbakterien lange Lebensdauern und Regenerationszyklen haben. Dies kann in der Bestimmung der zu ändernden Betriebskenngrößen berücksichtigt werden. Längere Zeitskalen sind von Bedeutung, wenn beispielsweise das Schlammalter zu hoch ist und Schlamm vermehrt abgeführt wird und durch neue Trockensubstanz für Belebtschlamm ersetzt wird. Es ist dann eine gewisse Zeit nötig, bis sich wieder stabile Verhältnisse in der Kläranlage 1 einstellen. Der in Fig. 2 dargestellte Zyklus von Messungen, Ermitteln des Betriebszustandes der Kläranlage 1, Veranlassungen von Änderung und Auswirkungen der Änderungen auf den Betriebszustand der Kläranlage 1 kann, je nach Einzelfall, Tage, Wochen oder Monate in Anspruch nehmen, bis ein optimaler Betriebszustand einer Kläranlage 1 eingestellt ist.

Das vorgestellte erfindungsgemäße Verfahren tritt an die Seite oder an die Stelle der Messung physikalisch-chemlscher Kenngrößen des Betriebszustands von biologischen Kläranlagen. Gerade durch die parallele Anwendung von mikrobiologischen und physikalisch-chemischen Messmethoden lässt sich ein sehr umfassendes Bild des Betriebszustandes von Kläranlagen erreichen, wobei die mikrobiologischen Messungen ein genaueres und differenziertes Bild der In der Kläranlage ablaufenden Prozesse erlauben.

Bezugszeichenliste

| 1 | Kläranlage |
|---|---|
| 2 | Belebtschlammbecken |
| 3 | Nachklärbecken |
| 4 | Zulauf |
| 5 | Überlauf |
| 6 | Ablauf |
| 7 | Belebtschlammrückführung |
| 8 | Rührer |
| 9 | Sauerstoffverteiler |
| 10 | Druckluftquelle |
| 11 | Abwasser-Belebtschlamm-Gemisch |
| 12 | Luftblasen |
| 13 | Fällmitteleintrag |
| 14 | Fällmitteleintrag |
| 15 | sedimentierter Belebtschlamm |
| 16 | gereinigtes Wasser |
| 20 | Messen von Häufigkeiten von Mikroorganismen im Beiebtschlamm |
| 21 | Ermitteln des Betriebszustandes der Kläranlage |
| 22 | Ändern von Betriebskenngrößen der Kläranlage |
| 30 | Ermitteln von Zustandskenngrößen |
| 31 | Ermitteln der notwendigen Änderungen von Betriebskenngrößen der Kläranlage |
| 32 | Auswirkungen der geänderten Betriebskenngrößen auf die Mikroorganismen im Belebtschlamm |

**Patentansprüche**

1. Verfahren zum Betreiben einer biologischen Kläranlage (1) mit wenigstens einem Belebtschlammbekken (2), **dadurch gekennzeichnet, dass** die Häufigkeit mehrerer Arten von im Belebtschlammbecken (2) enthaltenen Mikroorganismen gemessen wird und aus den Messergebnissen wenigstens eine Zustandskenngröße der Kläranlage (1) ermittelt wird.

2. Verfahren nach Anspruch 1, **dadurch gekennzeichnet, dass** zusätzlich wenigstens eine Kenngröße zur Beschaffenheit eines Inhalts des wenigstens einen Belebtschlammbeckens (2) gemessen wird.

3. Verfahren nach Anspruch 2, **dadurch gekennzeichnet, dass** als Kenngröße eine Farbe und/oder ein Geruch des Inhalts des wenigstens einen Belebtschlammbeckens (2) gemessen wird.

4. Verfahren nach Anspruch 2 oder 3, **dadurch gekennzeichnet, dass** als Kenngröße eine Opazität einer Wasserphase des Inhalts des wenigstens einen Belebtschlammbeckens (2) und/oder eine Häufigkeit frei lebender Bakterien in der Wasserphase gemessen wird.

5. Verfahren nach einem der Ansprüche 2 bis 4, **dadurch gekennzeichnet, dass** als Kenngröße eine Fädigkeit eines im wenigstens einen Belebtschlammbecken (2) enthaltenen Belebtschlammes und/oder eine Größe und/oder eine Struktur von Belebtschlammflocken des Belebtschlammes gemessen wird.

6. Verfahren nach einem der Ansprüche 1 bis 5, **dadurch gekennzeichnet, dass** als Zustandskenngröße eine Sauerstoffversorgung und/oder ein Sauerstoffversorgungsindex und/oder ein Saprobienindex und/oder eine Schlammbelastung und/oder eine Schadstoffbelastung und/oder eine Ablaufkonzentration und/oder eine Nitrifikationskenngröße ermittelt wird.

7. Verfahren nach einem der Ansprüche 1 bis 6, **dadurch gekennzeichnet, dass** zum Ermitteln von Zustandskenngrößen der Kläranlage (1) den Messergebnissen Gewichtungen, insbesondere für ihre jeweilige Indikatoreigenschaft für bestimmte Betriebszustände der Kläranlage (1), zugewiesen werden.

8. Verfahren nach Anspruch 7, **dadurch gekennzeichnet, dass** zum Ermitteln von Zustandskenngrößen der Kläranlage (1) die Produkte aus den Messergebnissen und den ihnen zugewiesenen Gewichtungen addiert werden.

9. Verfahren nach einem der Ansprüche 1 bis 8, **dadurch gekennzeichnet, dass** das Ermitteln von Zustandskenngrößen der Kläranlage (1) wiederholt wird.

10. Verfahren nach einem der Ansprüche 1 bis 9, **dadurch gekennzeichnet, dass** aufgrund wenigstens einer ermittelten Zustandskenngröße oder aufgrund einer Änderung wenigstens einer ermittelten Zustandskenngröße wenigstens eine Betriebskenngröße der Kläranlage (1) verändert wird.

11. Verfahren nach Anspruch 10, **dadurch gekennzeichnet, dass** als Betriebskenngröße der Kläranlage (1) eine Sauerstoffeintragung und/oder eine Eintragung einer Trockensubstanz in das wenigstens eine Belebtschlammbecken (2) und/oder eine Fällmittelzugabe (13, 14) in das wenigstens eine Belebtschlammbecken (2) und/oder in wenigstens ein dem wenigstens einen Belebtschlammbecken (2) nachgeschalteten Nachklärbekken (3) verändert wird.

12. Verfahren nach einem der Ansprüche 9 bis 11, **dadurch gekennzeichnet, dass** in aufeinanderfolgenden Iterationen des Ermittelns von Zustandsgrößen und/oder des Veränderns von Betriebskenngrößen der Kläranlage (1) die Gewichtung der Messergebnisse und/oder das Ausmaß der Veränderungen von Betriebskenngrößen, insbesondere In einem Selbstlernprozess, optimiert wird.

13. Verfahren nach einem der Ansprüche 1 bis 12, **dadurch gekennzeichnet, dass** die Messung der Häufigkeit wenigstens einer Art von im Belebtschlammbecken (2) enthaltenen Mikroorganismen und/oder die Messung einer weiteren Kenngröße zur Beschaffenheit eines Inhalts des wenigstens einen Belebtschlammbeckens (2) automatisch geschieht, Insbesondere mittels eines Bilderkennungsverfahrens.

14. Verfahren nach Anspruch 13, **dadurch gekennzeichnet, dass** das Bilderkennungsverfahren auf einem selbstiernfähigen Verfahren, insbesondere einem neuronalen Netz, basiert, das dazu trainiert wird, im Belebtschlammbecken (2) vorkommende Mikroorganismen zu identifizieren.

15. Computerprogramm mit Programmcode-Mitteln, die angepasst sind, um das Verfahren nach einem der Ansprüche 1 bis 14 auszuführen.

16. Computerprogramm nach Anspruch 15, gespeichert auf einem von einem Computer lesbaren Datenträger.

Fig. 1

Fig. 2

Europäisches
Patentamt

**EUROPÄISCHER RECHERCHENBERICHT**

Nummer der Anmeldung

EP 06 00 4586

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 38 11 097 A1 (ORPEGEN MEDIZINISCH-MOLEKULARBIOLOGISCHE FORSCHUNGSGESELLSCHAFT MBH, 6) 12. Oktober 1989 (1989-10-12) * Seite 2, Zeile 64 - Seite 3, Zeile 6; Ansprüche 1-7 * ----- | 1-16 | INV. C02F3/12 C02F3/30 |
| X,D | DE 42 44 708 C2 (MAIER, WERNER, 70372 STUTTGART, DE; VOLK, CHRISTIAN, 70499 STUTTGART,) 2. Mai 1996 (1996-05-02) * Spalte 1, Zeilen 3-47 * * Spalte 1, Zeile 64 - Spalte 2, Zeile 13; Ansprüche 1-24 * ----- | 1,15 | |
| X | DE 100 56 338 A1 (GEORG FRITZMEIER GMBH & CO) 7. Juni 2001 (2001-06-07) * Spalte 1, Zeilen 3-14 * * Spalte 5, Zeilen 39-55; Ansprüche 1,6 * ----- | 1,15 | |
| X | EP 0 461 166 B (AKTIESELSKABET FAXE KALKBRUD; WTE WASSERTECHNIK GMBH) 1. September 2004 (2004-09-01) * Absätze [0054] - [0057], [0062] - [0064], [0136], [0140], [0142], [0143], [0159]; Ansprüche 1-5,10,11 * ----- | 1,15 | RECHERCHIERTE SACHGEBIETE (IPC) C02F |
| X | WO 99/24370 A (BIOBALANCE A/S; NOERGAARD, PETER; MIKKELSEN, METTE, RISUM; HEINEN, NIC) 20. Mai 1999 (1999-05-20) * Seite 5, Zeilen 10-26; Ansprüche 1-3,7,10 * ----- | 1,15 | |
| X | US 5 906 746 A (HELMO ET AL) 25. Mai 1999 (1999-05-25) * Spalte 4, Zeilen 35-44 * * Spalte 6, Zeile 34 - Spalte 8, Zeile 67; Ansprüche 1-4; Abbildung 2 * ----- -/-- | 1,15 | |

Der vorliegende Recherchenbericht wurde für alle Patentansprüche erstellt

| Recherchenort | Abschlußdatum der Recherche | Prüfer |
|---|---|---|
| München | 15. Mai 2006 | Van Iddekinge, R |

EPO FORM 1503 03.82 (P04C03)

**Europäisches Patentamt**

# EUROPÄISCHER RECHERCHENBERICHT

Nummer der Anmeldung

EP 06 00 4586

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 5 401 412 A (YANG ET AL) 28. März 1995 (1995-03-28) * Zusammenfassung * * Spalte 5, Zeile 49 - Spalte 6, Zeile 37 * * Spalte 7, Zeile 59 - Spalte 8, Zeile 41; Ansprüche 7-9 * ----- | 1,15 | |
| X | US 5 324 431 A (WATANABE ET AL) 28. Juni 1994 (1994-06-28) * Spalte 6, Zeilen 9-68 * * Spalte 9, Zeile 15 - Spalte 10, Zeile 7; Ansprüche 1-6; Abbildungen 1,3 * ----- | 1,15 | |
| X | PATENT ABSTRACTS OF JAPAN Bd. 009, Nr. 153 (C-288), 27. Juni 1985 (1985-06-27) & JP 60 031886 A (HITACHI SEISAKUSHO KK), 18. Februar 1985 (1985-02-18) * Zusammenfassung * ----- | 1-16 | |
| X | PATENT ABSTRACTS OF JAPAN Bd. 018, Nr. 400 (C-1230), 26. Juli 1994 (1994-07-26) & JP 06 114391 A (NKK CORP), 26. April 1994 (1994-04-26) * Zusammenfassung * ----- | 1,15 | RECHERCHIERTE SACHGEBIETE (IPC) |
| X | PATENT ABSTRACTS OF JAPAN Bd. 1997, Nr. 08, 29. August 1997 (1997-08-29) & JP 09 096639 A (NGK INSULATORS LTD), 8. April 1997 (1997-04-08) * Zusammenfassung * ----- -/-- | 1,15 | |

Der vorliegende Recherchenbericht wurde für alle Patentansprüche erstellt

| Recherchenort | Abschlußdatum der Recherche | Prüfer |
|---|---|---|
| München | 15. Mai 2006 | Van Iddekinge, R |

EPO FORM 1503 03.82 (P04C03)

**Europäisches Patentamt**

**EUROPÄISCHER RECHERCHENBERICHT**

Nummer der Anmeldung

EP 06 00 4586

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | PATENT ABSTRACTS OF JAPAN Bd. 018, Nr. 142 (P-1706), 9. März 1994 (1994-03-09) & JP 05 322896 A (YAKULT HONSHA CO LTD; others: 01), 7. Dezember 1993 (1993-12-07) * Zusammenfassung * ----- | 1,15 | |
| X | PATENT ABSTRACTS OF JAPAN Bd. 1996, Nr. 12, 26. Dezember 1996 (1996-12-26) & JP 08 201298 A (HITACHI LTD), 9. August 1996 (1996-08-09) * Zusammenfassung * ----- | 1,15 | |
| X | PATENT ABSTRACTS OF JAPAN Bd. 2003, Nr. 12, 5. Dezember 2003 (2003-12-05) & JP 2004 008176 A (KURITA WATER IND LTD), 15. Januar 2004 (2004-01-15) * Zusammenfassung * ----- | 1,15 | |
| A | EP 0 410 877 A (VAN DEN HECKE, JEAN-CLAUDE; DUBOURG, ESTELLE) 30. Januar 1991 (1991-01-30) * Seite 8, Zeile 14 * ----- | 6 | **RECHERCHIERTE SACHGEBIETE (IPC)** |
| A | WALLEY WILLIAM J ET AL: "A reappraisal of saprobic values and indicator weights based on slovenian river quality data" WATER RESEARCH, ELSEVIER, AMSTERDAM, NL, Bd. 35, Nr. 18, Dezember 2001 (2001-12), Seiten 4285-4292, XP004320087 ISSN: 0043-1354 * Absatz [INTRODUCTION] * ----- | 6 | |

Der vorliegende Recherchenbericht wurde für alle Patentansprüche erstellt

| Recherchenort | Abschlußdatum der Recherche | Prüfer |
|---|---|---|
| München | 15. Mai 2006 | Van Iddekinge, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.** EP 06 00 4586

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-05-2006

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| DE 3811097 | A1 | | 12-10-1989 | AU | 3165089 | A | 05-10-1989 |
| | | | | CA | 1339959 | C | 21-07-1998 |
| | | | | DK | 157489 | A | 01-10-1989 |
| | | | | EP | 0336281 | A1 | 11-10-1989 |
| | | | | ES | 2037304 | T3 | 16-06-1993 |
| | | | | GR | 3004856 | T3 | 28-04-1993 |
| | | | | IL | 89765 | A | 25-01-1994 |
| | | | | JP | 2031892 | A | 01-02-1990 |
| | | | | NO | 891353 | A | 02-10-1989 |
| | | | | US | 5173187 | A | 22-12-1992 |
| DE 4244708 | C2 | | 02-05-1996 | KEINE | | | |
| DE 10056338 | A1 | | 07-06-2001 | KEINE | | | |
| EP 0461166 | B | | 01-06-1994 | AT | 106452 | T | 15-06-1994 |
| | | | | AU | 5199490 | A | 26-09-1990 |
| | | | | DE | 69009463 | D1 | 07-07-1994 |
| | | | | DE | 69009463 | T2 | 15-09-1994 |
| | | | | WO | 9010083 | A1 | 07-09-1990 |
| | | | | EP | 0461166 | A1 | 18-12-1991 |
| | | | | ES | 2054344 | T3 | 01-08-1994 |
| | | | | FI | 97240 | B | 31-07-1996 |
| | | | | NO | 913363 | A | 28-10-1991 |
| WO 9924370 | A | | 20-05-1999 | AU | 1144399 | A | 31-05-1999 |
| US 5906746 | A | | 25-05-1999 | AT | 174578 | T | 15-01-1999 |
| | | | | AU | 697064 | B2 | 24-09-1998 |
| | | | | AU | 5496696 | A | 29-11-1996 |
| | | | | BR | 9608119 | A | 21-09-1999 |
| | | | | CA | 2220680 | A1 | 14-11-1996 |
| | | | | DE | 69601178 | D1 | 28-01-1999 |
| | | | | DE | 69601178 | T2 | 22-07-1999 |
| | | | | WO | 9635644 | A1 | 14-11-1996 |
| | | | | DK | 0828692 | T3 | 23-08-1999 |
| | | | | EP | 0828692 | A1 | 18-03-1998 |
| | | | | ES | 2127014 | T3 | 01-04-1999 |
| | | | | GR | 3029427 | T3 | 28-05-1999 |
| | | | | JP | 11504854 | T | 11-05-1999 |
| | | | | NO | 975159 | A | 12-01-1998 |
| | | | | NZ | 306413 | A | 23-12-1998 |
| | | | | PL | 323258 | A1 | 16-03-1998 |
| | | | | ZA | 9603757 | A | 10-02-1998 |
| US 5401412 | A | | 28-03-1995 | KEINE | | | |

EPO FORM P0461

Für nähere Einzelheiten zu diesem Anhang : siehe Amtsblatt des Europäischen Patentamts, Nr.12/82

**EP 1 832 556 A1**

## ANHANG ZUM EUROPÄISCHEN RECHERCHENBERICHT
## ÜBER DIE EUROPÄISCHE PATENTANMELDUNG NR.

EP 06 00 4586

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-05-2006

| Im Recherchenbericht angeführtes Patentdokument | | Datum der Veröffentlichung | Mitglied(er) der Patentfamilie | | Datum der Veröffentlichung |
|---|---|---|---|---|---|
| US 5324431 | A | 28-06-1994 | JP<br>JP<br>JP | 2058790 C<br>3081645 A<br>7090234 B | 10-06-1996<br>08-04-1991<br>04-10-1995 |
| JP 60031886 | A | 18-02-1985 | JP<br>JP | 1775160 C<br>4062798 B | 28-07-1993<br>07-10-1992 |
| JP 06114391 | A | 26-04-1994 | KEINE | | |
| JP 09096639 | A | 08-04-1997 | JP | 3361216 B2 | 07-01-2003 |
| JP 05322896 | A | 07-12-1993 | KEINE | | |
| JP 08201298 | A | 09-08-1996 | JP | 3175518 B2 | 11-06-2001 |
| JP 2004008176 | A | 15-01-2004 | KEINE | | |
| EP 0410877 | A | 30-01-1991 | CA<br>DE<br>DE<br>ES<br>FR | 2028497 A1<br>69005387 D1<br>69005387 T2<br>2062447 T3<br>2650265 A1 | 25-04-1992<br>03-02-1994<br>28-07-1994<br>16-12-1994<br>01-02-1991 |

EPO FORM P0461

Für nähere Einzelheiten zu diesem Anhang : siehe Amtsblatt des Europäischen Patentamts, Nr.12/82

**EP 1 832 556 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4244708 C2